(19) Europäisches Patentamt
European Patent Office
Office européen des brevets

(11) Publication number: **0 060 081**
**B1**

(12) **EUROPEAN PATENT SPECIFICATION**

(45) Date of publication of patent specification: **17.07.85**

(51) Int. Cl.[4]: **C 07 D 239/95,**
A 61 K 39/385, A 61 K 39/395

(21) Application number: **82301068.1**

(22) Date of filing: **03.03.82**

(54) **Piperazinoquinazoline antihypertensives, processes for preparing them, pharmaceutical compositions containing them, and immunogenic protein conjugates formed from them.**

(30) Priority: **05.03.81 US 240745**

(43) Date of publication of application:
**15.09.82 Bulletin 82/37**

(45) Publication of the grant of the patent:
**17.07.85 Bulletin 85/29**

(84) Designated Contracting States:
**BE DE FR GB IT LU NL**

(56) References cited:
**EP-A-0 013 910**
**GB-A-2 008 106**

**Burger's Medicinal Chemistry, II, P. 308-9**

(73) Proprietor: **PFIZER INC.**
**235 East 42nd Street**
**New York, N.Y. 10017 (US)**

(72) Inventor: **Hess, Hans-Jurgen Ernst**
**Jericho Drive Old Lyme**
**New London Connecticut (US)**

(74) Representative: **Wood, David John et al**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 060 081 B1

## Description

The present invention relates to novel 1-(4-amino-6,7,8-substituted quinazolinyl)-4-substituted-piperazines and the homopiperazine analogs thereof which are useful as antihypertensive agents and as haptens in radioimmunoassays of related antihypertensive agents.

United States Patent No. 3,511,836 discloses a series of 2,4-diamino-6,7-dimethoxyquinazolines useful as antihypertensive agents, including 2-(4-substituted-piperazinyl) substituted compounds. Of particular interest as an antihypertensive agent has been prazosin, 2-(4-(2-furoyl)-piperazin-1-yl)-4-amino-6,7-dimethoxy-quinazoline. Similarly, United States Patent No. 3,669,968 discloses a series of 6,7,8-trialkoxy-2,4-diamino-quinazolines in which the 2-amino group is a heterocyclic group, including 4-substituted-piperazinyl. Of these compounds, a compound known by the generic name of trimazosin, 4-(4-amino-6,7,8-trimethoxyquinazolin-2-yl-piperazine-1-carboxylic acid-2-methyl-2-hydroxy-propyl ester is of particular interest. Homopiperazino-quinazoline analogs, useful as antihypertensive agents, are disclosed in United States Patent Nos. 3,920,631 and 4,044,135.

The general methods of radioimmunoassay are well known in the art. Such methods involve the formation of antibodies specific for a particular compound to be assayed. These antibodies are formed by innoculation of an animal with an appropriate immunogen. The antiserum containing the antibodies formed in response to the innoculation is then used to bind the compound to be measured, the measurement being based on a determination of the competitive binding of the antibodies with the compound and a radiolabeled derivative thereof. Calibration by use of solutions of known concentrations of the test compound allow quantitative determination of minute amounts of the test compound in animals in blood or other body fluids or tissues.

In general, most drugs, being relatively low molecular weight compounds, are not immunogenic per se. Such small molecules can, however, be rendered immunogenic by combination with a larger molecule, for example a protein, polypeptide or a polymer, having a molecular weight in excess of about 5,000.

The present invention relates to novel compounds useful as antihypertensive agents. More specifically, the present invention relates to compounds of the formula

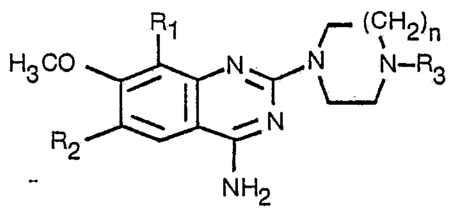

I

or a pharmaceutically acceptable salt thereof, wherein

$R_1$ is hydrogen or methoxy; $R_2$ is hydrogen, methoxy or chloro; n is 1 or 2; and $R_3$ is $-CO(CH_2)_a-X-(CH_2)_b-CO_2R$, wherein a and b are each zero or 1, X is $-CH_2-CH_2-$, $-CH{=}CH-$ or $-C{\equiv}C-$ and R is hydrogen or alkyl of 1 to 3 carbon atoms.

Preferred compounds are the piperazino analogs of formula I i.e. those compounds wherein n is 1. Preferred compounds include those wherein $R_1$ is hydrogen and $R_2$ is methoxy and where each of $R_1$ and $R_2$ are methoxy. Preferably, X is $-CH_2-CH_2-$ and, most preferably, a and b are each zero. R is preferably hydrogen.

Also embraced by the present invention are pharmaceutical compositions comprising an antihypertensive effective amount of a compound of formula I in combination with a pharmaceutically acceptable carrier or diluent. Preferred pharmaceutical compositions are those containing the preferred compounds of formula I, as described above.

The present invention also includes immunogenic protein conjugates comprising the condensation product of a compound of formula I and a protein having a molecular weight from about 5,000 to about 1,000,000, wherein the compound of formula I is bound to the protein by an amine bond formed by the reaction of the carboxyl group and a free amino group of the protein. Preferred protein conjugates are those containing the preferred compounds of formula I, as previously described, especially where the protein is Keyhole Limpet Hemocyanin or bovine albumin.

The invention further embraces a method of producing antibodies, specific for substituted quinazoline antihypertensive agents such as prazosin and trimazosin and related compounds, comprising parenterally administering to a mammal an antibody-producing effective amount of an immunogenic protein conjugate as described hereinabove, especially those containing the preferred compounds of formula I.

The compounds of formula I are readily prepared from a compound of the formula

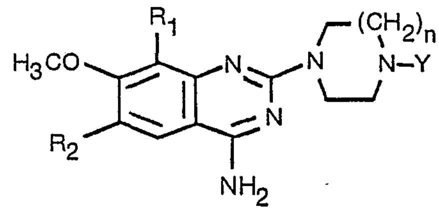

II

wherein Y is hydrogen. Such compounds may be prepared by the reaction of piperazine or homopiperazine with an appropriately substituted 2-halo-4-amino-quinazoline, which are known in the art and are readily prepared by the methods described in, for example, United States Patent Nos. 3,511,836, 3,669,968, 3,920,636 and 4,044,135 and which methods are summarized in copending application Serial No. 126,838 filed March 3, 1980.

The compounds of formula II where Y is hydrogen may be conveniently obtained, if desired, from an analog thereof wherein Y is a group which is subject to removal by hydrolysis. Such analogs include, for example, the 4-alkanoyl, 4-aroyl, 4-aralkoyl-piperazino derivatives and the 4-substituted-homopiperazino analogs thereof. Thus, for example, hydrolysis of the readily available compounds prazosin or trimazosin, effected for example by heating in the presence of a base such as an alkali metal hydroxide, at a temperature of from about 80°C to 120°C, will form the corresponding compound of formula II wherein Y is hydrogen and $R_1$ is hydrogen and $R_2$ is methoxy, or $R_1$ and $R_2$ are each methoxy, respectively.

The compound of formula II wherein Y is hydrogen is then reacted with an appropriate derivative of a dicarboxylic acid of the formula $HO_2C—(CH_2)_a—X—(CH_2)_b—CO_2H$. Suitable derivatives of the dicarboxylic acid include the anhydrides, acid halide-half esters and the like of, for example, succinic, glutaric, adipic, fumaric, maleic, acetylene dicarboxylic acids and analogs thereof as appropriate. The reaction is generally conducted at a temperature of from about 60°C to about 160°C, preferably from about 100°C to about 150°C, generally in an inert organic solvent such as an alkyl alcohol of 1 to 7 carbon atoms, preferably in isoamyl alcohol, or in a dialkyl ketone such as methyl ethyl ketone or ethyl isobutyl ketone.

In a further method of preparation, compounds of formula I may also be prepared according to methods described in United States Patent No. 3,511,836, for example by reaction of an appropriately substituted 2-halo-4-amino-quinazoline with a 4-$R_3$-piperazine or homopiperazine.

When obtained as the free acid i.e. when R is hydrogen, the compounds of formula I are readily converted, if desired, to the corresponding alkyl esters by conventional esterification methods, for example by heating with an appropriate alcohol or a diazolkane. Similarly, the alkyl esters of formula I are readily converted to the free acid by hydrolysis in the presence of a base in accord with conventional methods.

The present invention also includes the pharmaceutically acceptable salts of the compounds of formula I. Such salts include the acid addition salts with mineral or organic acids such as the acetate, or lactate, succinate, maleate, tartrate, citrate, gluconate, ascorbate, benzoate, fumarate, sulfate, phosphate, hydrochloride, tosylate, mesylate and the like. Further, for compounds wherein R is hydrogen, salts with pharmaceutically cations may be formed by conventional means. Such salts include the alkali metal salts, for example the sodium, potassium or lithium salts, and the alkaline earth metal salts, such as the calcium or magnesium salts.

The compounds of formula I and the pharmaceutically acceptable salts thereof are useful in the treatment of hypertension and in addition are useful in the treatment of other cardiovascular conditions such as angina, congestive heart failure or arrythmias. The compounds of the invention can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules either alone or in admixture with excipients, or in the form of elixirs or suspensions containing flavoring or coloring agents. They can be injected parenterally, for example, intramuscularly, intraveneously or subcutaneously. For parenteral administration, they are preferably used in the form of a sterile aqueous solution which may contain other solutes, for example, enough salt or glucose to make the solution isotonic. The compounds of the invention may be administered to animals for the treatment of hypertension or congestive heart failure or other cardiovascular conditions by either the oral or parenteral routes. For oral administration appropriate dosage amounts are from about 1 to about 20 mg/kg/day, given in a single dose or up to three divided doses. Thus, for an average human patient (70 kg) individual oral doses in tablet or capsule form will be approximately from about 70 to about 1400 mg of the active compound. Variations will necessarily occur depending on the weight and condition of the subject being treated and the particular route of administration chosen as will be well known to those skilled in the art. For parenteral administration, dosage amounts from about 0.4 to about 10 mg/kg/day may be employed.

The compounds of formula I are also useful as haptens for the generation of antibodies. Such antibodies, in turn, are useful, for example, in radioimmunoassays to monitor in patients' plasma levels of the well known antihypertensive agents prazosin and trimazosin and related compounds. Thus, the compounds of formula I, when combined with a suitable carrier molecule, such as a protein, polypeptide or polymer, will be immunogenic and will, when injected in an animal, produce antibodies necessary for the radioimmunoassay method. The ability of compounds of formula I to combine with appropriate carrier

molecules results from the presence of the terminal carboxy lgroup in the 4-piperazino- or homopiperazino substituent of the quinazoline ring, which may be reacted with free amino groups of a protein or a polypeptide molecule having a molecular weight generally from about 5,000 to 1,000,000 to form the desired immunogen, in accord with the general methods employed in the art.

As used in the specification and claims hereof the terms protein conjugate and condensation product are to be understood to mean the product formed by the reaction of the carboxyl group of a compound of formula I and free amino groups of a protein or polypeptide having a molecular weight from about 5,000 to 1,000,000, such that the compound of formula I is bonded to the protein molecule via an amide bond.

An appropriately substituted compound of formula I will be selected in view of the drug for which a radioimmunoassay is desired. Thus, for example, for the radioimmunoassay of prazosin, a compound of formula I wherein $R_1$ is hydrogen, $R_2$ is methoxy and n is 1 is most appropriate, preferably a compound where $R_3$ is succinoyl. Similarly, when a radioimmunoassay for trimazosin is of interest, a compound of formula I wherein $R_1$ and $R_2$ are each methoxy and n is 1 will be employed as the appropriate hapten. The compound of formula I as the free acid will generally be reacted with the free amino group of a suitable protein or polypeptide having a molecular weight from about 5,000 to 1,000,000, thereby linking the hapten of formula I to the carrier molecule by an amide bond. This reaction may be conveniently conducted in the presence of a carbodiimide, such as 1-ethyl-3(3-diethylaminopropyl)carbodiimide or N,N'-dicyclohexylcarbodiimide, in an inert organic solvent, such as dimethyl formamide, at a temperature from about 0 to 35°C, preferably about 15 to 25°C. While the type of protein or polypeptide employed is not critical and a variety of conventional protein sources may be employed, proteins such as Keyhole Limpet Hemocyanin (KLH) and bovine albumin may conveniently be employed. The immunogenic protein conjugate formed in the above manner is then isolated by conventional means, for example, by dialysis versus phosphate buffered saline solution.

The protein conjugate obtained as described above is then injected into a suitable animal such as a rabbit, sheep, goat or other mammal in an amount from about 0.1 mg/kg to about 1 mg/kg, followed by subsequent injections and, after an interval from about 2 to 6 weeks, the animal is bled to collect the antiserum. In a typical procedure, white New Zealand rabbits may be immunized by injections in each foot pad with about 0.4 mg of protein conjugate emulsified in complete Freunds adjuvant. A booster injection totalling about 1.0 mg of protein conjugate is made intramuscularly at multiple sites along the back about 4 weeks after the initial injection. After a further period of about 10 days the animals are bled to provide the antiserum.

The radioimmunoassay is carried out by using a radiolabeled form of a drug to be assayed, for example [$^3$H] prazosin or [$^3$H] trimazosin or a similar tritium labeled quinazoline derivative having antihypertensive activity. In a typical procedure, the radioimmunoassay may be carried out by mixing about 20,000 cpm of the radiolabeled drug with the antiserum. All solutions are diluted in an appropriate buffer solution having a pH in the range of about 7 to 8, preferably in a buffer solution containing 0.01 M Tris-HCl (pH 7.4), 0.15 M sodium chloride and 0.1 mg/ml gelatin. The mixture is incubated overnight at 4°C. Any drug which is not complexed to antibody is removed by adsorption on charcoal. Specifically, a suspension of activated charcoal in 0.1 M potassium phosphate buffer solution (pH 6.3) containing 5 mg/ml of bovine serum albumin is added to the assay tube. After about 20 minutes of incubation at 4°C, the charcoal is removed by centrifugation and the supernatant, representing the radioactivity bound to antibody, is assayed for radioactivity content. A standard curbe is then constructed in accordance with the well-known methodology of binding assays, as previously noted.

It will be understood that the methods described above are merely illustrative of the use of the compounds of formula I in radioimmunoassay methods and other procedures well known to those skilled in the art may be employed.

The present invention and preparation of certain starting materials is illustrated by the following Examples.

## Example 1

1-(4-Amino-6,7-dimethoxy-2-quinazolinyl)piperazine .75 Ethanol Solvate

100 g (0.24 mol) of 1-(4-amino-6,7-dimethoxy-2-quinazolinyl)-4-(2-furoyl) piperazine monohydrochloride (prazosin HCl) was suspended in a solution of 80 g (2.0 mol) sodium hydroxide in 800 ml water and heated at reflux for 168 hours. The reaction slurry was cooled, and a white solid was isolated by filtration, washed with water and dried in a vacuum oven at 60°C for 20 hours to give 79.8 g (115%) of a white solid, mp 229—232°C (with residual unmelted material). 65.0 g of this material was slurried and filtered from 2 × 800 ml water (pH filtrate 8.5), slurried in methylene chloride and dried *in vacuo* at 80°C for 20 hours to give 63.7 g, mp 225—227°C. This material was slurried repeatedly in 3 × 1L boiling ethanol and filtered to give a clear ethanol solution and 7.7 g of white solid, mp 300°C (ash content 91.99%). The clear ethanol solution was concentrated *in vacuo* to a total volume of 200 ml and cooled to 0°C. The resultant white solid was collected by filtration, washed with a small volume of ethanol followed by ether and was dried *in vacuo* at 23°C for 20 hours to give 57.3 g, mp 225—229°C (clear melt). NMR ($Me_2SO$) (δ): 7.36 (s, 1H); 6.97 (broad s, 2H); 6.68 (s, 1H); 3.85 (s, 3H); 3.82 (s, 3H); 3.63 (m, 4H); 3,47 (q, 0.75 X 2H—$C_2H_5OH$); 2.70 (m, 4H); 1.08 (t, 0.75 X 2H—$C_2H_5OH$).

Example 2

1-(4-Amino-6,7-dimethoxy-2-quinazolinyl)-4-succinoyl piperazine

14.5 g (0.0448 mol) 1-(4-amino-6,7-dimethoxy-2-quinazolinyl)piperazine.75 ethanol solvate was dissolved in 350 ml isoamyl alcohol at 100°C to give a clear solution. 5.0 g (0.05 mole) of succinic anhydride was added rapidly to give a clear solution. Within 1 minute a voluminous white precipitate began to form. The reaction was heated to reflux for 30 minutes and then cooled to 0°C, and the resultant white solid was collected by filtration and washed successively with isoamylalcohol, ethanol and ether. After air drying for 2 hours, the material was dried *in vacuo* at 60°C for 2 hours to give 16.8 g (96%) of a white solid mp 282°C with decomposition. NMR ($Me_2SO$) (δ): 9.90 (broad s, 1H); 7.43 (s, 1H), 7.10 (broad s, 2H): 6.76 (s, 1H); 3.90 (s, 3H); 3.87 (s, 3H); 3.70 (m, 8H); 2.57 (m, 4H); m/e calcd for $C_{18}H_{23}N_5O_5$: 389.1680; found 389.1677. Anal. calcd for $C_{18}H_{23}N_5O_5$: C, 55.52; H, 5.95; N, 17.98; found C, 55.31; H, 5.84; N, 17.62.

Example 3

1-(4-Amino-6,7,8-trimethoxy-2-quinazolinyl) piperazine hemihydrate

25.0 g (0.051 mol) 4-(4-Amino-6,7,8-trimethoxy-2-quinazolinyl)piperazine-1-carboxylic acid, 2-methyl-2-hydroxypropyl ester hydrochloride monohydrate (Trimazosin) was suspended in a solution of 20 g (0.5 mol) sodium hydroxide in 200 ml water and gradually brought to reflux. Considerable foaming was evident during the first hour of heating at reflux, but foaming gradually moderated after several hours and the reaction color changed from white to light pink. After 24 hours at reflux, the reaction color had changed to a light tan. The reaction was cooled to 23°C and the resultant solid was collected by filtration, washed thoroughly with water and dried in vacuo at 50°C overnight. The resultant solid was slurried in 200 ml chloroform and the clear filtrate was concentrated in vacuo and dried to give 12.3 g (73%) of 1-(4-amino-6,7,8-trimethoxy-2-quinazolinyl)piperazine hemihydrate: mp 187—190°C; NMR ($Me_2SO$) (δ): 7.25 (1H, s), 7.12 (2H, broad s), 4.0 (3H, s), 3,87 (6H, s), 3.65 (4H, m), 2.8 (4H, m). Anal. ($C_{15}H_{21}N_5O_3.\frac{1}{2}H_2O$) Calcd. C, 54.78; H, 6.32; N, 21.21. Found: C, 54.86; H, 6.75; N, 21.33.

Example 4

1-(4-Amino-6,7,8-trimethoxy-2-quinazolinyl)-4-succinoylpiperazine

1.06 g (3.23 mmol) 1-(4-Amino-6,7,8-trimethoxy-2-quinazolinyl)piperazine hemihydrate was dissolved in 20 ml of isoamyl alcohol and heated to reflux. 0.33 g (3.3 mmol) of succinic anhydride was added to give a clear solution. Within a few minutes a heavy white precipitate began to form. After 30 minutes heating at reflux, the reaction mixture was cooled to 0°C and the resultant solid was collected by filtration, washed with ethanol followed by ether and dried in vacuo at 70°C for 24 hours to give 1.27 g (94%) of 1-(4-amino-6,7,8-trimethoxy-2-quinazolinyl)-4-succinoylpiperazine: mp 253—245°C; NMR ($Me_2SO$) (δ): 7.30 (1H, s), 7.23 (2H, broad s), 4.0 (3H, s), 3.87 (6H, s), 3.73 (10H, m), 2.53 (2H, m). Anal. ($C_{19}H_{25}N_5O_6$) Calcd: C, 54.41; H, 6.01; N, 16.70. Found: C, 54.32; H, 5.80; N, 16.70.

**Claims**

1. A compound of the formula

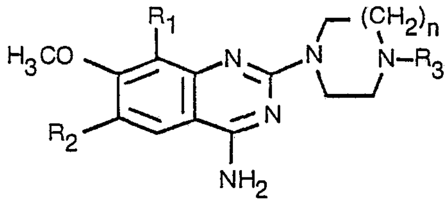

I

or a pharmaceutically acceptable salt thereof, wherein

$R_1$ is hydrogen or methoxy; $R_2$ is hydrogen, methoxy or chloro; n is 1 or 2; and $R_3$ is $—CO(CH_2)_a—X—(CH_2)_b—CO_2R$, wherein a and b are each independently zero or 1, X is $—CH_2—CH_2—$, $—CH=CH—$ or $—C\equiv C—$, and R is hydrogen or alkyl or 1 to 3 carbon atoms.

2. A compound according to claim 1 wherein n is 1.

3. A compound according to Claim 1 or 2 wherein X is $—CH_2—CH_2—$.

4. A compound according to any one of the preceding claims wherein $R_1$ is hydrogen or methoxy and $R_2$ is methoxy.

5. A compound according to claim 4 wherein a and b are each zero, R is hydrogen, X is $—CH_2CH_2—$, and n is 1.

6. A pharmaceutical composition comprising an antihypertensive effective amount of a compound according to any one of claims 1 to 5 and a pharmaceutically acceptable carrier.

7. An immunogenic protein conjugate comprising the condensation product of a compound according to any one of claims 1 to 5 and a protein having a molecular weight of from 5,000 to 1,000,000.

8. A protein conjugate according to claim 7 wherein in compound (I) n is 1, $R_1$ is hydrogen, $R_2$ is methoxy, X is $—CH_2—CH_2—$ and a and b are each zero.

9. A protein conjugate according to claim 7, wherein n is 1, $R_1$ and $R_2$ are each methoxy, X is $-CH_2-CH_2-$ and a and b are each zero.

10. A protein conjugate according to any one of claims 7 to 9 wherein the protein is Keyhole Limpet Hemocyanin or bovine albumin.

11. A method of producing antibodies, specific for substituted quinazoline antihypertensive agents such as prazosin and trimazosin, comprising parenterally administering to a mammal an antibody-producing amount of an immunogenic protein conjugate as defined in any one of claims 7 to 10.

12. A process for preparing a compound of the formula (I) as defined in claim 1, or a pharmaceutically acceptable salt thereof, characterized by reacting a compound of the formula:

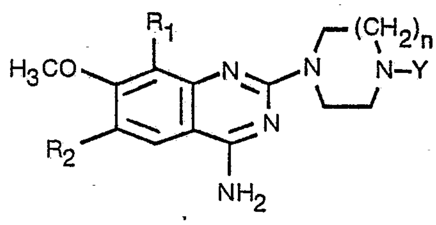
II

wherein $R_1$, $R_2$ and n are as defined in claim 1 and Y is hydrogen, with an acylating derivative of a dicarboxylic acid of the formula $HO_2C-(CH_2)_a-X-(CH_2)_b-CO_2H$, wherein a, b and X are as defined in claim 1, to yield the product of formula (I) and thereafter if required, (i) converting the compound of formula (I) into a pharmaceutically acceptable salt thereof by reaction with a non-toxic acid or appropriate cation when R is H, or (ii) converting a free acid product of formula (I) where R is hydrogen to a lower alkyl ester thereof where R is alkyl of 1 to 3 carbon atoms by esterification, or (iii) converting a product of the formula (I) in which R is $C_1-C_3$ alkyl into a product of the formula (I) in which R is H by hydrolysis.

**Patentansprüche**

1. Verbindung der Formel

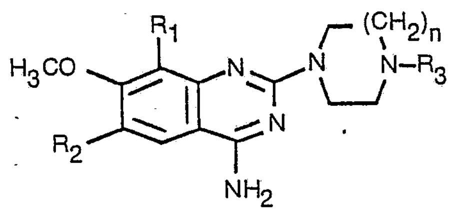
I

oder ein pharmazeutisch annehmbares Salz davon, wobei $R_1$ Wasserstoff oder eine Methoxygruppe ist; $R_2$ Wasserstoff, eine Methoxygruppe oder Chlor ist; n 1 oder 2 ist; und $R_3$ $-CO(CH_2)_a-X-(CH_2)_b-CO_2R$ ist, wobei a und b jeweils unabhängig voneinander 0 oder 1 sind, X $-CH_2-CH_2-$, $-CH=CH-$ oder $-C\equiv C-$ ist und R Wasserstoff oder ein Alkylrest mit 1 bis 3 Kohlenstoffatomen ist.

2. Verbindung gemäß Anspruch 1 wobei n gleich 1 ist.

3. Verbindung gemäß Anspruch 1 oder 2 wobei X $-CH_2-CH_2-$ ist.

4. Verbindung gemäß einem der vohergehenden Ansprüche, wobei $R_1$ Wasserstoff oder eine Methoxygruppe und $R_2$ eine Methoxygruppe ist.

5. Verbindung gemäß Anspruch 4, wobei a und b jeweils 0 sind, R Wasserstoff ist, C $-CH_2CH_2-$ ist und n gleich 1 ist.

6. Pharmazeutische Zusammensetzung im wesentlichen bestehend aus einer antihypertonisch wirksamen Menge einer Verbindung gemäß einem der Ansprüche 1 bis 5 und einem pharmazeutisch annehmbaren Träger.

7. Immunogenes Proteinkonjugat, das das Kondensationsprodukt aus einer Verbindung gemäß einem der Ansprüche 1 bis 5 und einem Protein mit einem Molekulargewicht zwischen 5000 und 1 000 000 beinhaltet.

8. Proteinkonjugat gemäß Anspruch 7, wobei in der Verbindung (I) n gleich 1 ist, $R_1$ Wasserstoff ist, $R_2$ eine Methoxygruppe ist, X $-CH_2-CH_2-$ ist und a und b jeweils 0 sind.

9. Proteinkonjugat gemäß Anspruch 7, wobei n gleich 1 ist, $R_1$ und $R_2$ jeweils Methoxygruppen sind, X $-CH_2-CH_2-$ ist und a und b jeweils 0 sind.

10. Proteinkonjugat gemäß einem der Ansprüche 7 bis 9, wobei das Protein Keyhole Limpet-Hemocyanin oder Rinderalbumin ist.

11. Verfahren zur Herstellung von Antikörpern, die spezifisch für substituierte Chinazolin antihypertonische Wirkstoffe wie Prazosin und Trimazosin sind, wobei einem Säugetier parenteral eine Antikörper produzierende Menge eines immunogenen Proteinkonjugates gemäß einem der Ansprüche 7 bis 10 verabreicht wird.

12. Verfahren zur Herstellung einer Verbindung der Formel (I) gemäß Anspruch 1 oder eines pharmazeutisch annehmbaren Salzes davon, dadurch gekennzeichnet, daß man eine Verbindung der Formel:

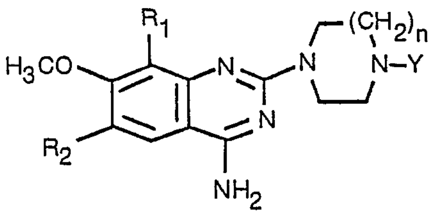

II

wobei $R_1$, $R_2$ udn n der Definition in Anspruch 1 entsprechen und Y Wasserstoff ist, mit einem acylierenden Derivat einer Dicarbonsäure der Formel $HO_2C—(CH_2)_a—X—(CH_2)_b—CO_2H$, wobei a, b und X der Definition in Anspruch 1 entsprechen, zu dem Produkt der Formel (I) umsetzt und anschließend gegebenenfalls, (i) eine Verbindung der Formel (I) in ein pharmazeutisch annehmbares Salz davon durch Reaktion mit einer nicht-toxischen Säure oder einem geeigneten Kation, wenn R gleich H ist, umwandelt oder (ii) ein in Form der freien Säure vorliegendes Produkt der Formel (I), worin R Wasserstoff ist, durch Veresterung zu einem niederen Alkylester davon, worin R ein Alkylrest mit 1 bis 3 Kohlenstoffatomen ist umwandelt oder (iii) ein Produkt der Formel (I), in der R ein $C_1—C_3$-Alkylrest ist durch Hydrolyse in ein Produkt der Formel (I) in der R = H ist, umwandelt.

**Revendications**

1. Composé de formule

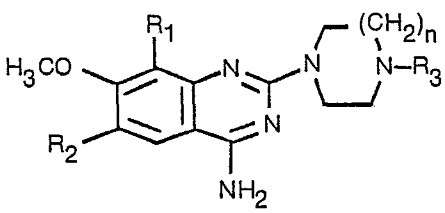

I

ou sel pharmaceutiquement acceptable de ce composé, formule dans laquelle

$R_1$ est l'hydrogène ou le groupe méthoxy; $R_2$ est l'hydrogène, le groupe méthoxy ou le radical chloro; *n* est égal à 1 ou 2; et $R_3$ est un groupe de formule $—CO(CH_2)_a—X—(CH_2)_b—CO_2R$, dans laquelle *a* et *b* représentent chacun, indépendamment, 0 ou 1, X est un groupe $—CH_2—CH_2—$, $—CH=CH—$ ou $—C{\equiv}C—$, et R est l'hydrogène ou un groupe alkyle ayant 1 à 3 atomes de carbone.

2. Composé suivant la revendication 1, dans lequel *n* est égal à 1.

3. Composé suivant la revendication 1 ou 2, dans lequel X est un groupe $—CH_2—CH_2—$.

4. Composé suivant l'une quelconque des revendications précédentes, dans lequel $R_1$ est l'hydrogène ou le groupe méthoxy et $R_2$ est le groupe méthoxy.

5. Composé suivant la revendication 4, dans lequel *a* et *b* sont tous deux égaux à zéro, R est l'hydrogène, X est un groupe $—CH_2CH_2—$ et *n* est égal à 1.

6. Composition pharmaceutique comprenant une quantité à effet antihypertensif d'un composé suivant l'une quelconque des revendications 1 à 5 et un support pharmaceutiquement acceptable.

7. Conjugué de protéine immunogène, comprenant le produit de condensation d'un composé suivant l'une quelconque des revendications 1 à 5 et d'une protéine ayant un poids moléculaire de 5000 à 1 000 000.

8. Conjugué de protéine suivant la revendication 7, dans lequel, dans le composé (I), *n* est égal à 1, $R_1$ est l'hydrogène, $R_2$ est le groupe méthoxy, X est un groupe $—CH_2—CH_2—$ et *a* et *b* sont tous deux égaux à zéro.

9. Conjugué de protéine suivant la revendication 7, dans lequel *n* est égal à 1, $R_1$ et $R_2$ sont chacun un groupe méthoxy, X est le groupe $—CH_2—CH_2—$ et *a* et *b* sont tous deux égaux à zéro.

10. Conjugué de protéine suivant l'une quelconque des revendications 7 à 9, dans lequel la protéine est l'hémocyanine de Fissurella ou l'albumine de boeuf.

11. Procédé de production d'anticorps, spécifiques d'agents antihypertensifs quinazoliniques subsitués tels que prazosine et trimazosine, consistant à administrer par voie parentérale à un mammifère une quantité génératrice d'anticorps d'un conjugué de protéine immunogène tel que défini dans l'une quelconque des revendication 7 à 10.

12. Procédé de préparation d'un composé de formule (I) telle que définie dans la revendication 1 ou d'un sel pharmaceutiquement acceptable de ce composé, caractérisé par la réaction d'un composé de formule:

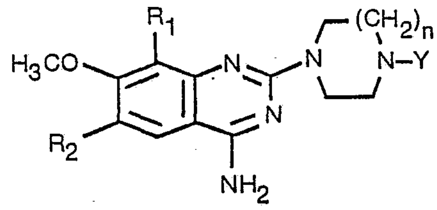

II

dans laquelle $R_1$, $R_2$ et *n* sont tels que définis dans la revendication 1 et Y est l'hydrogène, avec un dérivé acylant d'un acide dicarboxylique de formule $HO_2C—(CH_2)_a—X—(CH_2)_b—CO_2H$, dans laquelle *a*, *b* et X sont tels que définis dans la revendication 1, pour obtenir le produit de formule (I) puis, le cas échéant, (i) la transformation du composé de formule (I) en un sel pharmaceutiquement acceptable de ce composé par réaction avec un acide non toxique ou un cation approprié lorsque R représente H, ou (ii) transformation d'un produit acide libre de formule (I) dans laquelle R est l'hydrogène en un ester alkylique inférieur de ce produit où R est un groupe alkyle ayant 1 à 3 atomes de carbone par estérification, ou (iii) transformation d'un produit de formule (I) dans laquelle R est un groupe alkyle en $C_1$ à $C_3$ en un produit de formule (I) dans laquelle R représente H, par hydrolyse.